# EUROPEAN PATENT SPECIFICATION

(11) **EP 0 932 387 B1**
(45) Date of publication and mention of the grant of the patent: **05.03.2003**
(21) Application number: 97910315.7
(22) Date of filing: 18.09.1997
(51) Int. Cl.: A61K 7/48

(54) **SKIN CARE COMPOSITIONS CONTAINING COMBINATIONS OF COMPOUNDS FOR MIMICKING THE EFFECT ON SKIN OF RETINOIC ACID**
HAUTPFLEGEMITTEL ENTHALTEND KOMBINATIONEN AUS VERBINDUNGEN DIE DIE WIRKUNGVON RETINSAÜRE AUF DER HAUT NACHBILDEN
COMPOSITIONS POUR LE SOIN DE LA PEAU CONTENANT DES COMBINAISONS DE COMPOSES PERMETTANT DE REPRODUIRE L'EFFET SUR LA PEAU DE L'ACIDE RETINOIQUE

(30) Priority: 27.09.1996 US 26824 P
(43) Date of publication of application: 04.08.1999
(73) Proprietor: UNILEVER PLC, London EC4P 4BQ (GB); UNILEVER N.V., 3013 AL Rotterdam (NL)
(72) Inventor: GRANGER, Stewart, Paton, Paramus, NJ 07652 (US); BURGER, Allan, Robert, Passaic, NJ 07055 (US); SCOTT, Ian, Richard, Allendale, NJ 07401 (US); IWATA, Koichi, Ridgefield Park, NJ 07660 (US); ZHANG, Kelly, Hua, Piscataway, NJ 08854 (US); RAWLINGS, Anthony, Vincent, Northwich,Cheshire CW9 8FH (GB)
(74) Representative: Rots, Maria Johanna Francisca
(86) International application number: EP9705150
(87) International publication number: WO98013020

(56) References cited:
- EP-A- 0 601 698
- EP-A- 0 608 433
- WO-A-96/03973
- FR-A- 2 558 058
- FR-A- 2 666 226
- US-A- 5 536 740
- US-A- 5 665 367
- DATABASE WPI Week 9601 Derwent Publications Ltd., London, GB; AN 96-008675 XP002055760 & RO 109 503 B (BUZAN ET AL.)

## Description

### Field of the Invention

The present invention relates to skin care compositions containing certain compounds in combination with retinol and/or retinyl ester and to cosmetic methods involving applying such compositions to the skin.

### Background of the Invention

Retinol (vitamin A) is an endogenous compound which occurs naturally in the human body and is essential for normal epithelial cell differentiation. Natural and synthetic vitamin A derivatives have been used extensively in the treatment of a variety of skin disorders and have been used as skin repair or renewal agents. Retinoic acid has been employed to treat a variety of skin conditions, e.g., acne, wrinkles, psoriasis, age spots and discoloration. See e.g., Vahlquist, A. et al., *J. Invest. Dermatol.,* Vol. 94, Holland D.B. and Cunliffe, W.J. (1990), pp. 496-498; Ellis, C.N. et al., "Pharmacology of Retinols in Skin", Vasel, Karger, Vol. 3, (1989), pp. 249-252; Lowe, N.J. et al., "Pharmacology of Retinols in Skin", Vol. 3, (1989), pp. 240-248; PCT Patent Application No. WO 93/19743.

It is believed that the use of retinol or esters of retinol would be preferred over retinoic acid. Retinol occurs naturally in the human body and is considered much safer than retinoic acid. Esters of retinol hydrolyze in-vivo to produce retinol. It is believed that retinol esters and retinol are metabolically converted in the skin into retinoic acid according to the following mechanism:

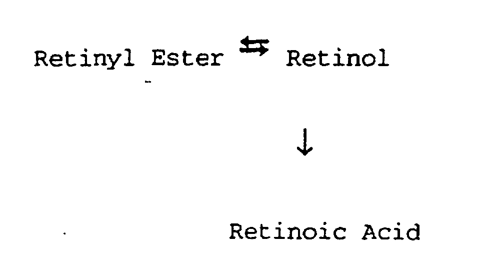

However, most of the endogenously applied retinol is rapidly converted into inactive fatty esters for storage in epidermal cells (keratinocytes). Esterification of retinol into inactive retinyl esters is achieved in cells by transfer of a fatty acyl group from an acyl CoA, catalyzed by the enzyme acyl CoA retinol transferase (ARAT), or by the transfer of an acyl group from phosphatidyl choline, catalyzed by the enzyme lecithin retinol acyl transferase (LRAT). These esterification reactions are very efficient in keratinocytes- the majority (95%) of cellular retinoids are in the form of retinyl fatty esters. Thus, unfortunately, although retinol and retinyl esters are safer to use than retinoic acid, they are less effective than retinoic acid at providing skin benefits.

The present invention is based, in part, on the discovery that certain compounds inhibit these esterification reactions and thus potentiate the action of retinol by increasing the amount of retinol available for conversion to retinoic acid. Thus, a mixture of these compounds with retinol or retinyl esters mimics retinoic acid yet is safer to use than retinoic acid.

### Summary of the Invention

The present invention includes, in part, a skin conditioning composition containing:
(a) from 0.001% to 10% of a ccmpound selected from the group consisting of retinol, retinyl ester and mixtures thereof;
(b) from 0.0001% to 50% of a compound which at 100 µM concentration inhibits at least 20% of LRAT or ARAT catalyzed retinol esterification as measured by an in vitro Microsomal Assay as described herein; wherein the compound is not a fatty acid amide or dimethyl imidazolidinone and is selected from the group consisting of cyclic aliphatic unsaturated hydrocarbons, diterpenes, and fatty hydroxyethyl imidazoline surfactants having the following structure: 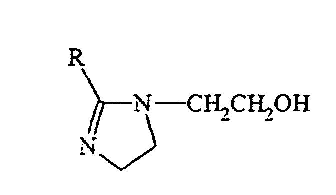 wherein R is an aliphatic saturated or unsaturated, straight or branched hydro-carbon chain containing from 3 to 20 carbon atoms; and mixtures thereof; and
(c) a cosmetically acceptable vehicle.

The compounds included in the present invention in combination with retinol and/or retinyl ester are selected based on the ability of such combinations to mimic retionoic acid's effect on skin. Put another way, if the compound inhibits sufficiently on LRAT or ARAT catalyzed retinol esterification as measured by an in-vitro Microsomal Assay, it will act in combination with retinol or retinyl ester to mimic the effect on keratinocytes (skin cells) of retinoic acid.

The invention further provides a cosmetic method of conditioning skin comprising topically applying the present composition to the skin. The invention also provides a cosmetic method mimicking the effect of retinoic acid on skin, comprising topically applying the present composition to the skin.

The term "conditioning" as used herein means prevention and treatment of one or more of the following: dry skin, photodamaged skin, appearance of wrinkles, age spots, aged skin. The compositions are also useful for increasing stratum corneum flexibility, lightening skin color, controlling sebum excretion and generally increasing the quality of skin. The composition may be used to improve skin desquamation and epidermal differentiation.

The presence of the selected compounds in the inventive composition substantially improves the performance of retinol or retinyl ester.

According to the present invention, by virtue of including an effective amount of a compound which at 100 µM concentration inhibits at least 20% of ARAT or LRAT catalyzed retinol esterification as measured by in vitro Microsomal Assay, into compositions containing retinol or a retinyl ester, the performance of the compositions is substantially improved.

### Description of the Preferred Embodiment

The inventive compositions contain, as a first essential ingredient, a compound selected from the group consisting of retinol and retinyl ester. The term "retinol" includes amongst others the following isomers of retinol: all-trans-retinol, 13-cis-retinol, 11-cis-retinol, 9-cis-retinol, 3,4-didehydro-retinol. Preferred isomers are all-trans-retinol, 13-cis-retinol, 3,4-didehydro-retinol, 9-cis-retinol. Most preferred is all-trans-retinol, due to its wide commercial availability.

Retinyl ester is an ester of retinol. The term "retinol" has been defined above. Retinyl esters suitable for use in the present invention are C₁-C₃₀ esters of retinol, preferably C₂-C₂₀ esters, and most preferably C₂, C₃, and C₁₆ esters because they are more commonly available. Examples of retinyl esters include but are not limited to: retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecandate, retinyl laurate, retinyl tridecanoate, retinyl myristate, retinyl pentadecanoate, retinyl heptadeconoate, retinyl stearate, retinyl isostearate, retinyl nonadecanoate, retinyl arachidonate, retinyl behenate, retinyl linoleate, retinyl oleate.

The preferred ester for use in the present invention is selected from retinyl palmitate, retinyl acetate and retinyl propionate, because these are the most commercially available and therefore the cheapest. Retinyl linoleate is also preferred due to its efficacy.

Retinol and/or retinyl ester is employed in the inventive composition in an amount of from 0.001% to 10%, preferably in an amount of from 0.01% to 1%, most preferably in an amount of from 0.01% to 0.5%.

The second essential ingredient of the inventive compositions is a compound which passes an in vitro Microsomal Assay. A compound suitable for use in the present invention inhibits at 100 µM concentration, at least 20% of LRAT or ARAT catalyzed retinol esterification as measured by in vitro Microsomal Assay. The in vitro Microsomal Assay employed for determining the suitability of the inclusion of the compound in the inventive compositions is as follows:

In a preferred embodiment of the invention, a compound is selected which, at a 100 µM concentration, inhibits at least 40% and most preferably at least 50%, of LRAT or ARAT catalyzed retinol esterification.

### In vitro Microsomal Assay:

Microsomes are obtained as described in: J.C. Saari and D.L. Bredberg, "CoA and Non-CoA Dependent Retinol Esterification in Retinal Figment Epithelium" J. Biol. Chem. 263, 8084-90 (1988).

A solution containing 0.1M sodium phosphate pH 7 buffer, 5mM dithiothreitol, 2 mg/ml bovine serum albumin, 40 micromolar palmitoyl CoA, 40 micromolar dilauroyl phosphatidyl choline, 10 micromolar retinol and a test compound or a solvent blank, is incubated for 1 hour at 37°C with a microsomal fraction isolated from bovine retinal pigment epithelial cells. After incubation, the reaction is quenched by addition of an equal volume of ethanol, and the retinyl esters formed (retinyl laurate from the LRAT catalyzed reaction and retinyl palmitate from ARAT catalyzed reaction) are extracted with hexane. The hexane layer is removed, evaporated under nitrogen, and the residue analyzed by HPLC on a 3.9x300 mm C₁₉ reversed phase column using a 80% methanol in tetrahydrofuran mobile phase and fluorescence detection (325 nm excitation, 480 nm emission) to quantitate the retinyl ester. The quantity of ester formed in the presence of the solvent blank is taken as 100%, and this is used to calculate the percent inhibition of ester formation for the compounds tested. As a control, an aliquot of microsomes is inactivated by boiling for 5 minutes, which results in at least 95% inhibition of ester formation.

Cyclic aliphatic unsaturated hydrocarbons, diterpenes, and certain fatty hydroxyethyl imidazoline surfactants are examples of the compounds which satisfy the in-vitro Microsomal Assay test described above. These compounds are described individually hereinbelow. Of course, other compounds not explicitly mentioned herein are included in the inventive compositions, as long as they pass the in-vitro Microsomal Assay Test described herein.

### Cyclic Aliphatic Unsaturated Compounds

Suitable cyclic aliphatic unsaturated compounds are selected according to the in-vitro Microsomal Assay Test described above.

A preferred cyclic aliphatic unsaturated compound is selected from cyclic aliphatic unsaturated aldehydes, ketones, alcohols and esters.

Preferred cyclic aliphatic unsaturated aldehydes, ketones, alcohols and esters are: alpha damascone, beta damascone, delta damascone, isodamascone, damascenone, alpha ionone, beta ionone, allyl alpha ionone, isobutyl ionone, alpha methyl ionone, gaga methyl ionone, brahmanol, sandanol, alpha terpineol, lyral, ethyl saffranate, and mixtures thereof. Structures of these compounds are as follows:

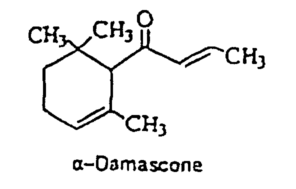

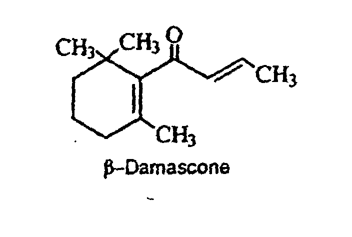

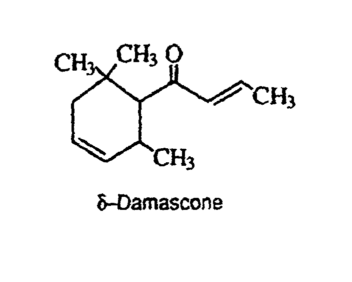

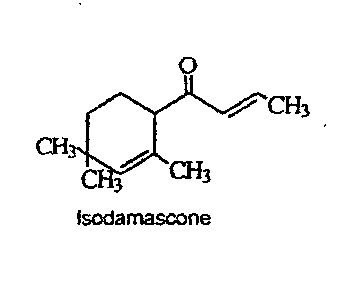

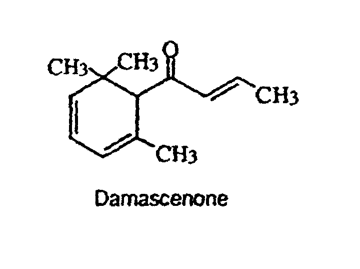

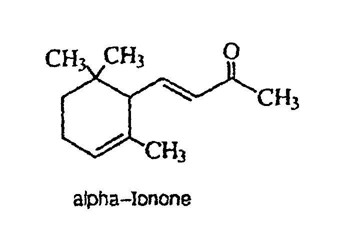

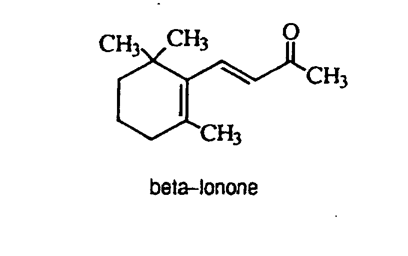

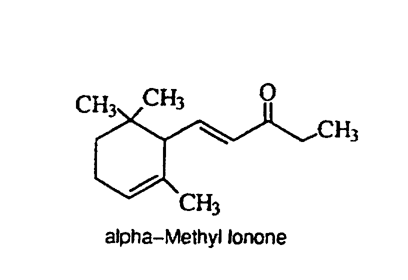

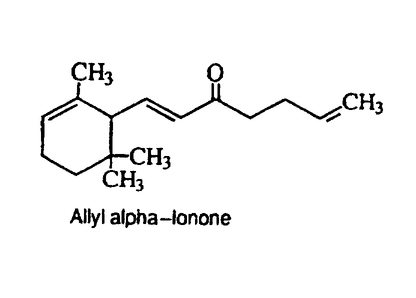

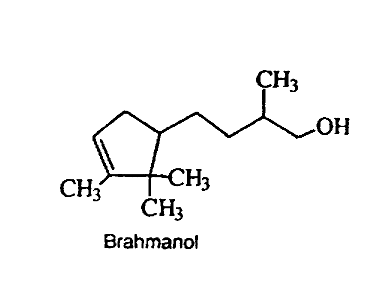

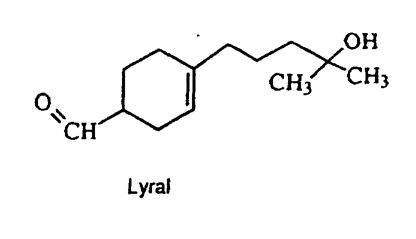

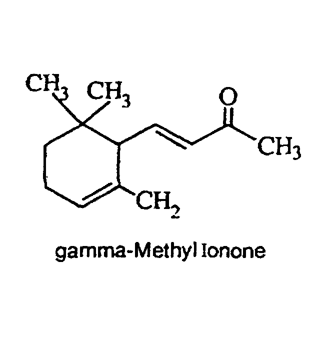

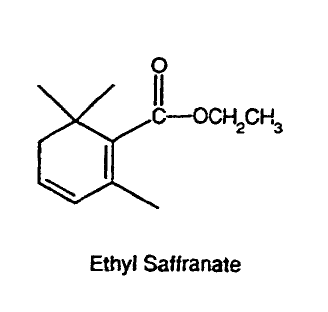

Preferably, in order to maximize performance at a minimum cost, a cyclic aliphatic unsaturated compound is selected from the group consisting of damascones and ionones having the structures described above.

Most preferably, the cyclic aliphatic unsaturated compound is a α-Damascone and/or α-lonone.

The cyclic aliphatic unsaturated compound can be included in the inventive compositions in an amount ranging from 0.0001% to 50% by weight of the composition, preferably it is used in an amount of from 0.01% co 10%, most preferably from 0.1% to 5%.

### Diterpenes

Diterpenes suitable for inclusion in the present invention are those that pass the in-vitro Microsomal Assay described hereinabove. A preferred diterpene compound is geranyl geraniol, which has the following structure:

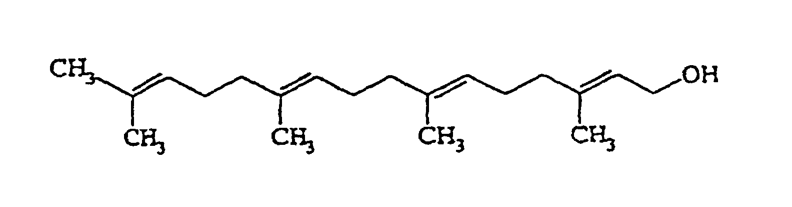

Diterpene can be included in the inventive compositions in an amount ranging from 0.0001% to 50%, preferably it is used in an amount of from 0.01% to 10%, most preferably from 0.1% to 5%.

The structures of naringenin and quercetin are as follows:

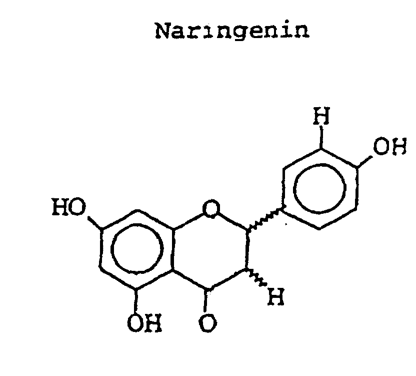

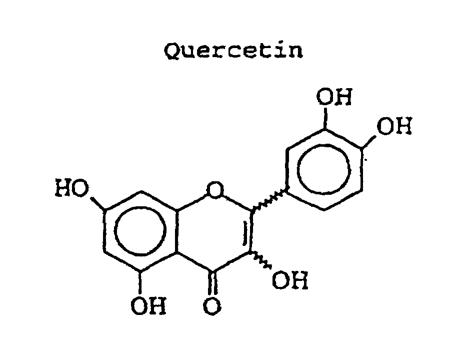

The flavonone or flavonol can be included in the inventive compositions in an amount ranging from 0.0001% to 50%, preferably it is used in an amount of from 0.01% to 10%, most preferably from 0.1% to 5%.

Quercetin and/or naringenin may be obtained from Sigma. Plant extracts containing quercetin and/or naringenin are also suitable for use in the present invention, e.g. rutin, evening primrose, onion, citrus species.

### Fatty Hydroxethyl Imidazoline Surfactants

Fatty hydroxyethyl imidazoline surfactants included in the present invention pass the in-vitro Microsomal Assay test described above. Preferred fatty hydroxyethyl imidazolines have the following general structure: 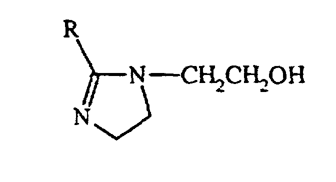 wherein R is an aliphatic saturated or unsaturated, straight or branched hydro-carbon chain containing from 8 to 20 carbon atoms.

Examples of suitable fatty hydroxyethyl imidazolines include but are not limited to cocoylhydroxyethyl imidazoline (R is derived from coconut oil fatty acids, mostly C₁₂ and some longer chain), lauryl hydroxyethyl imidazoline (R=CH₃(CH₂)₁₀), myristyl hydroxyethyl imidazoline (R=CH₃(CH₂)₁₂), stearyl hydroxyethyl imidazoline (R=CH₃(CH₂)₁₆), and oleyl hydroxyethyl imidazoline (R=CH₃(CH₂)₇ CH=CH(CH₂)₇).

Preferably, R in the fatty hydroxyethyl imidazoline contains from 8 to 18 carbon atoms, more preferably from 11 to 18 carbon atoms. Most preferably, the fatty hydroxyethyl imidazoline is oleyl hydroxyethyl imidazoline, due to its commercial availability and efficacy.

The fatty hydroxyethyl imidazoline can be included in the inventive compositions in an amount ranging from 0.0001% to 50%, preferably it is used in an amount of from 0.01% to 10%, most preferably from 0-1% to 5%.

### Cosmetically Acceptable Vehicle

The composition according to the invention also comprises a cosmetically acceptable vehicle to act as a dilutant, dispersant or carrier for the active ingredients in the composition, so as to facilitate their distribution when the composition is applied to the skin.

Vehicles other than or in addition to water can include liquid or solid emollients, solvents, humectants, thickeners and powders. An especially preferred nonaqueous carrier is a polydimethyl siloxane and/or a polydimethyl phenyl siloxane. Silicones of this invention may be those with viscosities ranging anywhere from 10 to 10,000,000mm^{2/}s(centistokes) at 25°C. Especially desirable are mixtures of low and high viscosity silicones. These silicones are available from the General Electric Company under trademarks Vicasil, SE and SF and from the Dow Corning Company under the 200 and 550 Series. Amounts of silicone which can be utilized in the compositions of this invention range anywhere from 5% to 95%, preferably from 25% to 90% by weight of the composition.

The cosmetically acceptable vehicle will usually form from 5% to 99.9%, preferably from 25% to 80% by weight of the composition, and can, in the absence of other cosmetic adjuncts, form the balance of the composition. Preferably, the vehicle is at least 50 wt.%, more preferably at least 80 wt.% water, by weight of the vehicle. Preferably, water comprises at least 50 wt.% of the inventive composition, most preferably from 60 to 80 wt.%, by weight of the composition.

### Optional Skin Benefit Materials and Cosmetic Adjuncts

An oil or oily material may be present, together with an emulsifier to provide either a water-in-oil emulsion or an oil-in-water emulsion, depending largely on the average hydrophilic-lipophilic balance (HLB) of the emulsifier employed.

The inventive compositions preferably include sunscreens. Sunscreens include those materials commonly employed to block ultraviolet light. Illustrative compounds are the derivatives of PABA, cinnamate and salicylate. For example, octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively. The exact amount of sunscreen employed in the emulsions can vary depending upon the degree of protection desired from the sun's UV radiation.

Another preferred optional ingredient is selected from essential fatty acids (EFAs), i.e., those fatty acids which are essential for the plasma membrane formation of all cells, in keratinocytes EFA deficiency makes cells hyperproliferative. Supplementation of EFA corrects this. EFAs also enhance lipid biosynthesis of epidermis and provide lipids for the barrier formation of the epidermis. The essential fatty acids are preferably chosen from linoleic acid, γ-linolenic acid, homo-γ-linolenic acid, columbinic acid, eicosa-(n-6,9,13)-trienoic acid, arachidonic acid, α-linolenic acid, timnodonic acid, hexaenoic acid and mixtures thereof.

Emollients are often incorporated into cosmetic compositions of the present invention. Levels of such emollients may range from 0.5% to 50%, preferably between 5% and 30% by weight of the total composition. Emollients may be classified under such general chemical categories as esters, fatty acids and alcohols, polyols and hydrocarbons.

Esters may be mono- or di-esters. Acceptable examples of fatty di-esters include dibutyl adipate, diethyl sebacate, diisopropyl dimerate, and dioctyl succinate. Acceptable branched chain fatty esters include 2-ethyl-hexyl myristate, isopropyl stearate and isostearyl palmitate. Acceptable tribasic acid esters include triisopropyl trilinoleate and trilauryl citrate. Acceptable straight chain fatty esters include lauryl palmitate, myristyl lactate, oleyl eurcate and stearyl oleate. Preferred esters include coco-caprylate/caprate (a blend of coco-caprylate and coco-caprate), propylene glycol myristyl ether acetate, diisopropyl adipate and cetyl octanoate.

Suitable fatty alcohols and acids include those compounds having from 10 to 20 carbon atoms. Especially preferred are such compounds such as cetyl, myristyl, palmitic and stearyl alcohols and acids.

Among the polyols which may serve as emollients are linear and branched chain alkyl polyhydroxyl compounds. For example, propylene glycol, sorbitol and glycerin are preferred. Also useful may be polymeric polyols such as poly-propylene glycol and polyethylene glycol. Butylene and propylene glycol are also especially preferred as penetration enhancers.

Exemplary hydrocarbons which may serve as emollients are those having hydrocarbon chains anywhere from 12 to 30 carbon atoms. Specific examples include mineral oil, petroleum jelly, squalene and isoparaffins.

Another category of functional ingredients within the cosmetic compositions of the present invention are thickeners. A thickener will usually be present in amounts anywhere from 0.1 to 20% by weight, preferably from 0.5% to 10% by weight of the composition. Exemplary thickeners are cross-linked polyacrylate materials available under the trademark Carbopol from the B.F. Goodrich Company. Gums may be employed such as xanthan, carrageenan, gelatin, karaya, pectin and locust bean gum. Under certain circumstances the thickening function may be accomplished by a material also serving as a silicone or emollient. For instance, silicone gums with viscosity in excess of 10 centistokes and esters such as glycerol stearate have dual functionality.

Powders may be incorporated into the cosmetic composition of the invention. These powders include chalk, talc, kaolin, starch, smectite clays, chemically modified magnesium aluminum silicate, organically modified montmorillonite clay, hydrated aluminum silicate, fumed silica, aluminum starch octenyl succinate and mixtures thereof.

Other adjunct minor components may also be incorporated into the cosmetic compositions. These ingredients may include coloring agents, opacifiers and perfumes. Amounts of these other adjunct minor components may range anywhere from 0.001% up to 20% by weight of the composition.

### Use of the Composition

The composition according to the invention is intended primarily as a product for topical application to human skin, especially as an agent for conditioning and smoothening the skin, and preventing or reducing the appearance of wrinkled or aged skin.

In use, a small quantity of the composition, for example from 1 to 100ml, is applied to exposed areas of the skin, from a suitable container or applicator and, if necessary, it is then spread over and/or rubbed into the skin using the hand or fingers or a suitable device.

### Product Form and Packaging

The topical skin treatment composition of the invention can suitably be formulated as a lotion, a cream or a gel. The composition can be packaged in a suitable container to suit its viscosity and intended use by the consumer. For example, a lotion or cream can be packaged in a bottle or a roll-ball applicator, or a propellant-driven aerosol device or a container fitted with a pump suitable for finger operation. When the composition is a cream, it can simply be stored in a non-deformable bottle or squeeze container, such as a tube or a lidded jar.

The composition may also be included in capsules such as those described in U.S. Patent 5,063,057.

The invention accordingly also provides a closed container containing a cosmetically acceptable composition as herein defined.

The following specific examples further illustrate the invention.

### MATERIALS AND METHODS

### Cell Culture:

Human keratinocytes, isolated from neonatal foreskin by trypsin treatment were grown in Dulbecco Modification Eagle (DME) Hams F12 (1:1) medium/10% fetal calf serum in the presence of irradiated 3T3 mouse fibroblasts for establishing dividing keratinocyte colonies. Cells were grown under the above condition until their second passage and kept frozen for future use. Frozen second passage keratinocytes were thawed and plated into the above medium and grown for five days before they were switched to a serum-free MCDB 153-based medium keratinocyte growth medium (KGM) from Clonetics Corporation, San Diego, CA, containing 0.15 mM Ca, or keratinocyte serum-free media (KSFM) from GIBCO containing 0.09 mM Ca). On day 7, when the cells were 80-90% confluent, they were trypsinized and plated in the serum-free medium for the various experiments.

### Thymidine Assay

### ³H-Thymidine Incorporation and Keratinocyte Proliferation

The incorporation of ³H-thymidine by cultured keratinocytes was used as an assay of keratinocyte proliferation. Thymidine is one of four deoxynucleosides which are the monomeric units of DNA, the universal library of genetic information in the animal kingdom. Prior to cell division of a somatic cell such as a keratinocyte, the complete genome of the cell undergoing cell division is replicated. This involves large scale DNA synthesis by the cell and enables both daughter cells to receive identical copies of the genetic material. When ³H-thymidine is included in the culture media of keratinocytes which are synthesizing DNA in preparation for cell division then the labelled nucleoside is incorporated into the newly synthesized DNA. The extent of incorporation of ³H-thymidine into a population of cells is proportional to the rate of DNA synthesis by this population of cells and therefore an indication of their cellular proliferation.

Keratinocytes (that were cultured as described above) were plated in 24 well plates at a density of 20,000 cells per well in 1 ml media. After incubation for four days or until the cells were 60-70% confluent, the media was changed. Test compounds were added (in triplicate) to the wells 24 hours after the media change, and four hours later 1µCi ³H-Thymidine in 50 µl media was added per well. Cells were incubated for a further 24 hours. Media was removed from the cells, 10% ice cold trichloroacetic acid (TCA) added and plates were incubated on ice for 30 minutes. Cells were washed five times with 5% TCA and allowed to dissolve in 500 µl 0.1M NaOH for at least one hour (usually overnight). The preparations were neutralized with 0.1M HCl; 50 µl of the cell preparation was used to determine total protein content. Disintegrations per minute (DPM) from ³H labelling of DNA was determined by liquid scintillation counting of 900µl of the cell preparation. Thymidine incorporation results were expressed as DPM/µg protein.

### Transglutaminase Assay

### Transglutaminase Assay and Keratinocyte Differentiation

During the process of terminal differentiation in the epidermis, a 15nm thick layer of protein, known as the cornified envelope (CE) is formed on the inner surface of the cell periphery. The CE is composed of numerous distinct proteins which have been cross-linked together by the formation of N^{ε}-(γ-glutamyl) lysine isodipeptide bonds catalyzed by the action of at least two different transglutaminases (TGases) expressed in the epidermis. Transglutaminase I (TGase I) is expressed in abundance in the differentiated layers of the epidermis, especially the granular layer, but is absent in the undifferentiated basal epidermis. Thus TGase I is a useful marker of epidermal keratinocyte differentiation with high TGase I levels indicating a more differentiated state. An ELISA based TGase I assay, using a TGase I antibody, was used to assess the state of differentiation of the cultured keratinocytes in the examples that follow.

For Example 1, the following procedure was used:

Keratinocytes (cultured as described above) were plated in 96 well plates at a density of 3,000 cells per well in 200 µl media. After incubation for four days the media was changed to media containing test compounds (six replicates per test). The cells were cultured for a further 72 hours after which time the media was aspirated and the plates stored at -70°C. Plates were removed from the freezer, and the cells washed with PBS. 100 µl sterile water was added and the cells were freeze fractured by freezing at -70°C then thawing. The cells were incubated for one hour at room temperature (R/T) with PBS/3% BSA (wash buffer, bovine serum albumin), then rinsed with a fresh aliquot of wash buffer. Cells were incubated with 50 µl of primary antibodies monoclonal anti-human transglutaminase mouse antibody (IgG) obtained from Biomedical Industries diluted 1:2,000 in wash buffer for one hour, 37°C then rinsed two times with wash buffer. Cells were then incubated with 50 µl of secondary antibody (Fab fragment, peroxidase conjugated anti-mouse IgG obtaining from Amersham) diluted 1:4,000 in wash buffer for one hour at 37°C, then rinsed two times with wash buffer. Cells were incubated with substrate solution (4 mg o-phenylene diamine and 3.3 µl 30% H₂O₂ in 10ml 0.1M citrate buffer pH 5.0) for five minutes, R/T, in darkness (under aluminum foil). The reaction was stopped by the addition of 50 µl 4N H₂SO₄. The absorbance of samples was read at 492nm in the plate reader. Out of the six replicates, four were treated with both antibodies, two were treated only with the secondary antibody (i.e., to determine background binding of enzyme conjugated Ab). TGase levels were determined by subtracting background from the readings from each treatment and determining mean ± s.d. for the replicates exposed to both antibodies.

For other Examples in which TGase I level was measured, the following procedure was used:

Keratinocytes (cultured as described above) were plated in 96 well plates at a density of 3,000 cells per well in 200µl of cell culture media. After incubation for four days, the media was changed to media containing test compounds (six replicates per test). The cells were cultured for a further 72 hours after which time the media was aspirated and the plates stored at -70°C. After the plates were removed from the freezer, the cells were further freezed fractured by freezing and thawing and then washed 3x with PBS. The cells were incubated for one hour at room temperature (R/T) with TBS/5% BSA buffer. Cells were then incubated with 100µl of monoclonal anti-human transglutaminase (IgG) mouse antibody (primary antibody) obtained from Biomedical Technologies Inc. diluted 1:2000 in TBS/1% BSA buffer for two hours at 37°C, and then rinsed six times with wash buffer (TBS/1% BSA/0.05% Tween-20). Cells were next incubated with 100µl of Fab fragment, peroxidase conjugated anti-mouse IgG antibody (secondary antibody) from Amersham diluted 1:4,000 in wash buffer for two hours at 37°C and then rinsed three times with wash buffer and three times with PBS. Cells were incubated with substrate solution (4mg o-phenylene diamine and 3.3µl 30% H₂O₂ in 10mL 0.1M citrate buffer, pH 5.0) for five minutes at R/T and in darkness (under aluminum foil). The reaction was stopped by the addition of 50µl 4N H₂SO₄. The absorbance of samples was read at 492nm in the plate reader. Out of the six replicates, four were treated with both antibodies, two were treated only with the secondary antibody (i.e., to determine the background binding of the enzyme conjugated antibody). Transglutaminase I levels were determined by subtracted background from the readings from each treatment and determining the mean ± s.d. for the replicates exposed to both antibodies.

### DNA Assay

The level of TGase I detected after treatment of the cells could be influenced by cell number, i.e., the greater the number of cells the greater the level of TGase-1 detected. The level of Tgase I was normalized to DNA content of the cells in the same well thus eliminating variation due to differences in cell number. DNA quantitation is a particularly useful indicator of cell number, including keratinocyte cell number, because each cell has to all intents and purposes an identical genome and therefore an identical quantity of DNA. The total DNA content of a well of cells therefore is directly proportional to the cell number in that well. Quantitation of DNA was used to normalize the TGase data to cell number.

Keratinocytes were plated in 96 well plates at a density of 3,000 cells per well in 200µl media. After incubation for four days the media was changed for media containing-test compounds (6 replicates per test). The cells were cultured for a further 72 hours after which time the media was aspirated and the plates stored for at least 1.5 hours at -70°C. Plates were removed from the freezer and thawed for 30 minutes. 100µl of Hoechst dye (1µg/ml final concentration) was added and this was incubated for 15 minutes, covered and then read in a fluorimeter (ex. 360nm and em. 460nm). The dye solution was removed and the wells were rinsed with PBS in preparation for the TGase assay.

### EXAMPLE 1

### Retinoic acid is more effective than retinol at altering keratinocyte differentiation state

The effect on Transglutaminase levels normalized to DNA content of the cells after addition of retinoic acid (RA) and retinol (ROH) was examined- and the results are shown in Table 1.

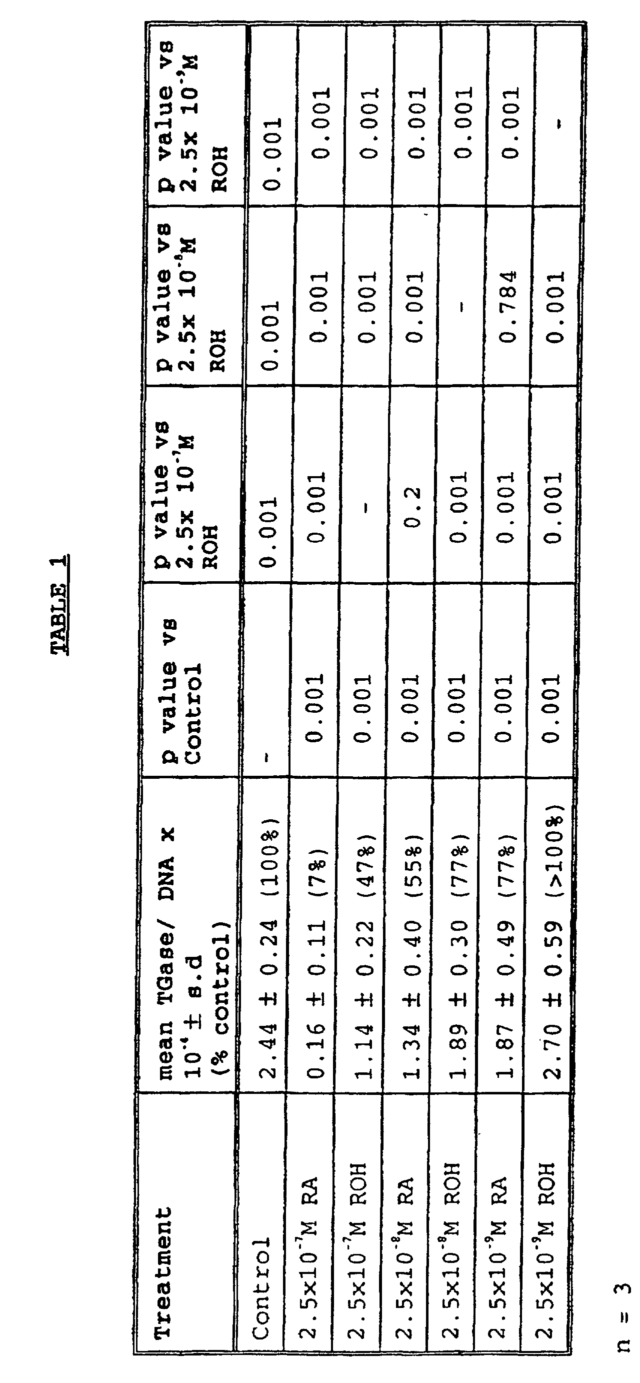

All concentrations of retinoic acid tested, i.e., 2.5 x 10⁻⁷M, 2.5 x 10⁻⁸M and 2.5x10⁻⁹M decreased keratinocyte differentiation over the ethanol control and did so to a significantly greater extent than each of the corresponding 2.5x10⁻⁷M, 2.5x10⁻⁸M and 2.5x10⁻⁹M retinol treatments. The decrease in transglutaminase level was dose dependent for both retinoic acid and retinol. This is consistent with retinoic acid having a greater inhibitory effect on epithelial differentiation than retinol.

### EXAMPLE 2

### Method of in vitro microsomal esterification of retinol:

Microsomes are obtained as described in: J.C. Saari and D.L. Bredberg, "CoA and Non-CoA Dependent Retinol Esterification in Retinal Pigment Epithelium" J. Biol. Chem. 23, 8084-90 (1988).

A solution containing 0.1M sodium phosphate pH 7 buffer, 5mM dithiothreitol, 2 mg/ml bovine serum albumin, 40 micromolar palmitoyl CoA, 40 micromolar dilauroyl phosphatidyl choline, 10 micromolar retinol and a test compound or solvent blank, was incubated for 1 hour at 37°C with a microsomal fraction isolated from bovine retinal pigment epithelial cells. After incubation, the reaction was quenched by addition of an equal volume of ethanol, and the retinyl esters formed (retinyl palmitate from the ARAT catalyzed reaction, and retinyl laurate from the LRAT catalyzed reaction) were extracted with hexane. The hexane layer was removed, evaporated under nitrogen, and the residue analyzed by HPLC on a 3.9x300 mm C18 reversed phase column using a 80% methanol in tetrahydrofuran mobile phase and fluorescence detection (325 nm excitation, 480 nm emission) to quantitate the retinyl esters. The quantity of ester formed in the presence of the solvent blank was taken as 100%, and this was used to calculate the percent inhibition of ester formation for the compounds tested. As a control, an aliquot of microsomes was inactivated by boiling for 5 minutes, which resulted in at least 95% inhibition of ester formation.

The results that were obtained are summarized in Table 2.

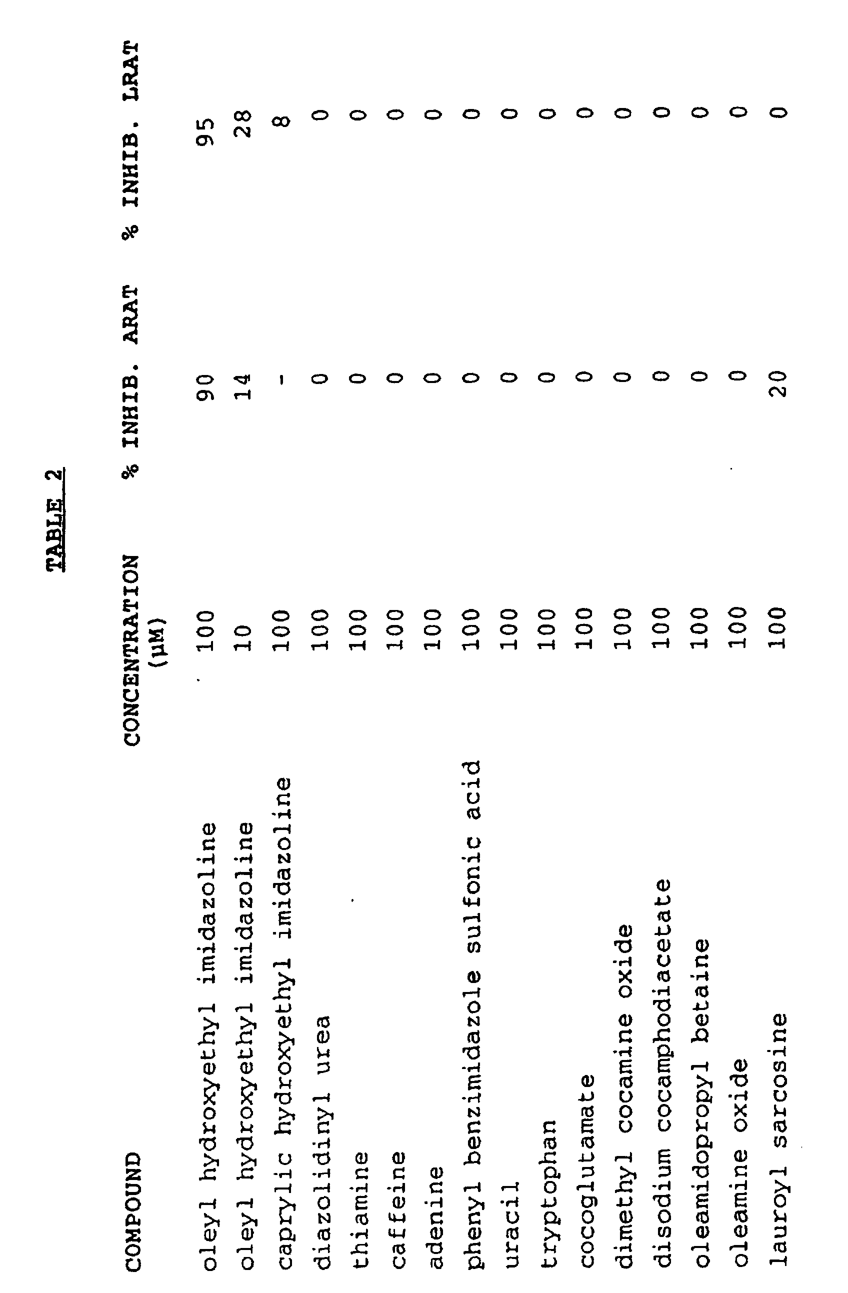

It can be seen that the hydroxyethyl imidazoline surfactant is a potent esterification inhibitor, while other surfactants and other heterocyclic compounds were essentially inactive. Caprylic hydroxyethyl imidazoline (R=CH₃(CH₃)₆) did not sufficiently inhibit LRAT.

### EXAMPLE 3

Example 2 was repeated with various hydroxyethyl imidazoline surfactants, having R groups as indicated in Table 3. The results that were obtained are summarized in Table 3.

**TABLE 3**

| **R GROUP** | **CONCENTRATION (MICROMOLAR)** | **% INHIB. ARAT** | **% INHIB. LRAT** |
|---|---|---|---|
| Oleyl | 100 | 90 | 90 |
| Oleyl | 10 | 15 | 12 |
| Lauryl | 100 | 53 | 47 |
| Lauryl | 10 | 0 | 0 |
| Coco | 100 | 68 | 65 |
| Coco | 10 | 0 | 0 |

The results in Table 3 show relative activity oleyl>coco>lauryl. Coco is better probably because it is not purely C₁₂, but has some longer chain alkyl groups as well.

The data in Table 2 on caprylic hydroxyethyl imidazoline showed only 8% inhibition at 100 micromolar. Taken together the data clearly shows relative activity oleyl>coco>lauryl>>caprylic.

### EXAMPLE 4

Transglutaminase Assay was conducted using oleyl hydroxyethyl imidazoline (OHI), retinoic acid (RA) , retinol (ROH), and retinyl palmitate (RP).

The results that were obtained are summarized in Table 4.

**TABLE 4**

| **Exp#** | **Compound(s)** | **Concentration (Micromolar)** | **% of Control** |
|---|---|---|---|
| 1 | RA | 0.25 | 35.1 |
| 1 | ROH | 0.25 | 76.2 |
| 1 | OHI | 1 | 80.7 |
| 1 | ROH+OHI | 0.25+1 | 45.1 |
| | | | |
| 2 | RA | 0.25 | 43.4 |
| 2 | ROH | 0.025 | 91.6 |
| 2 | OHI | 1 | 78.2 |
| 2 | ROH+OHI | 0.025+1 | 57.9 |
| | | | |
| 3 | RA | 0.25 | 45.9 |
| 3 | RP | 0.25 | 91.6 |
| 3 | OHI | 1 | 91.1 |
| 3 | RP+OHI | 0.25+1 | 71.6 |

It can be seen from the results in Table 4 that retinoic acid substantially decreased keratinocyte differentiation whereas retinol, oleyl hydroxyethyl imidazoline, and retinyl palmitate did not affect keratinocyte differentiation when used alone, or did so to a much smaller degree than retinoic acid.

In experiments 1 and 2, when retinol was combined with oleyl hydroxyethyl imidazoline, the result was a synergistic decrease in keratinocyte differentiation, to levels approaching the effect of retinoic acid.

In experiment 3, when oleyl hydroxyethyl imidazoline was combined with retinyl palmitate, the result was not as dramatic as in experiments 1 and 2, but nevertheless a synergistic decrease was observed.

This example also confirms that a compound which passed the in-vitro Microsomal Assay described herein, actually had an effect on keratinocytes when combined with retinol at a level similar to the effect of retinoic acid.

### EXAMPLE 5

The in vitro Microsomal Assay Test was run on the compounds listed in Tables 5A and 5B.

The compounds in Table 5A were tested at a 100µM concentration. The compounds in Table 5B were tested at a 10µM concentration.

**TABLE 5A**

| **COMPOUND** | **% INHIBITION, ARAT** | **% INHIBITION, LRAT** |
|---|---|---|
| alpha damascone | 83 | 98 |
| beta damascone | 84 | 92 |
| delta damascone | 87 | 95 |
| isodamascone | 80 | 92 |
| damascenone | 70 | 79 |
| alpha ionone | 45 | 49 |
| beta ionone | 22 | 24 |
| allyl alpha ionone | 22 | 36 |
| isobutyl ionone | 8 | 45 |
| alpha methyl ionone | 67 | 77 |
| gamma methyl ionone | 21 | 38 |
| brahmanol | 70 | 75 |
| sandanol | 15 | 43 |
| alpha terpineol | 26 | 25 |
| timberol | 34 | 33 |
| lyral | 76 | 71 |
| tonalid | 50 | 33 |
| ethyl saffranate | 51 | 49 |
| traseolide | 41 | 21 |
| sandalone | 23 | 12 |

**TABLE 5B**

| **COMPOUND** | **% INHIBITION, ARAT** | **% INHIBITION, LRAT** |
|---|---|---|
| alpha damascone | 67 | 87 |
| beta damascone | 45 | 52 |
| delta damascone | 58 | 64 |
| damascenone | 23 | 29 |
| allyl alpha ionone | 16 | 17 |

It can be seen from the results in Tables 5A and 5B that certain cyclic aliphatic unsaturated compounds are potent inhibitors of LRAT and ARAT catalyzed retinol esterification.

### COMPARATIVE EXAMPLE 6

The in-vitro Microsomal Assay test was conducted with additional cyclic aliphatic unsaturated compounds. The results that were obtained are summarized in Table 6.

The compounds in Table 6 were tested at a 100 µM concentration.

**TABLE 6**

| **COMPOUND** | **% INHIBITION, ARAT** | **% INHIBITION, LRAT** |
|---|---|---|
| dihydro alpha ionone | 13 | 18 |
| alpha ionol | 0 | 0 |
| beta ionol | 0 | 0 |
| cinnamaldehyde | 0 | 0 |
| vanillin | 0 | 0 |
| eucalyptol | 0 | 0 |
| menthol | 0 | 0 |
| thymol | 0 | 0 |
| carvone | 0 | 0 |
| camphor | 0 | 0 |
| mentone | 0 | 0 |
| fenchyl alcohol | 12 | 4 |
| isocyclogeraniol | 18 | 16 |
| dimethyl ionone | 0 | 9 |
| delta methyl ionone | 0 | 10 |

It can be seen from the results in Table 6 that not all cyclic aliphatic unsaturated compounds inhibit or sufficiently inhibit LRAT and ARAT catalyzed retinol esterification.

### EXAMPLE 7

The effect on keratinocyte differentiation of compounds and combinations listed in Table 7 was examined. The results were expressed as % of control. Transglutaminase level was normalized to DNA. Data are from two experiments where the retinol concentration was changed. The results that were obtained are summarized in Table 7.

**TABLE 7**

| **EXPERIMENT #** | **TREATMENT** | **CONCENTRATIONmM** | **% CONTROL** |
|---|---|---|---|
| 1 | Retinoic acid | 0.00025 | 24 |
| 1 | Retinol | 0.001 | 67 |
| 1 | α-Damascone | 1 | 92 |
| 1 | Retinol + α-Damascone | 0.001 + 1 | 34 |
| 2 | Retinoic acid | 0.00025 | 8 |
| 2 | Retinol | 0.00025 | 63.5 |
| 2 | α-Damascone | 1 | 86 |
| 2 | Retinol + α-Damascone | 0.00025 + 1 | 30 |

The results in Table 7 show that while α-Damascone alone and retinol alone were not very effective, the combination of the two attained synergistic reduction in transglutaminase mimicking the effect of retinoic acid on keratinocyte differentiation. This example also establishes a good correlation between the Microsomal Assay and cell culture data.

### EXAMPLE 8

The in-vitro Microsomal Assay test was conducted with a diterpene compound, geranyl geraniol or farnesol.

The results that were obtained are summarized in Table 8.

**TABLE 8**

| **COMPOUND** | **CONCENTRATION (µM)** | **% INHIB. ARAT** | **% INHIB. LRAT** |
|---|---|---|---|
| Geranyl Geraniol¹ | 100 | 81 | 77 |
| Geranyl Geraniol | 10 | 38 | 16 |
| Farnesol² | 100 | 43 | 43 |
| Farnesol | 10 | 20 | 10 |

| | | | |
|---|---|---|---|
| ¹ Obtained from TCI America (Portland,Oregon). Also available from Sigma and CTC Organics (Atlanta, Georgia). | | | |
| ² Available from Givaudan Co., Bedoukian Co., or Dragoco Co. | | | |

It can be seen from the results in Table 8 that both geranyl geraniol and farnesol inhibit retinol esterification. Geranyl geraniol is a substantially more potent esterification inhibitor, than farnesol, the structure for which is as follows:

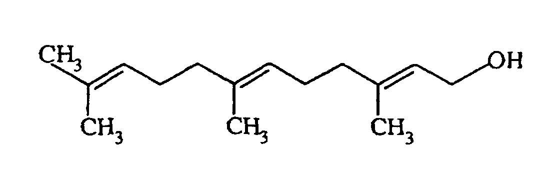

### EXAMPLE 9

³H-Thymidine incorporation was measured according to the procedure described in Materials and Methods section above. The results that were obtained are summarized in Tables 9A and 9B.

**TABLE 9A**

| **Experiment #1** | | | |
|---|---|---|---|
| **Treatment** | **DPM/Microgram Protein** | | **% Control** |
| | mean | (s.d.) | |
| Control | | (278) | 100 |
| 250 nM retinol | 2082 | (146) | 108 |
| 250 nM retinoic acid | 3013 | (226) | 156 |
| 250nM retinol + 10 nM geranyl geraniol | 2970 | (308) | 154 |

**TABLE 9B**

| **Experiment #2** | | | |
|---|---|---|---|
| **Treatment** | **DPM/Microgram Protein** | | **% Control** |
| | mean | (s.d.) | |
| Control | | (322) | 100 |
| 250 nM retinol | 974 | (148) | 107 |
| 250 nM retinoic acid | 1494 | (45) | 163 |
| 250nM retinol + 10 nM geranyl geraniol | 1450 | (318) | 159 |

It can be seen from the results above that geranyl geraniol is able to significantly increase the proliferation enhancing effect of retinol to a level similar to that which can be achieved with a comparable amount of retinoic acid. Again, a good correlation is proven to exist between the Microsomal Assay test and the effect of a compound on cell culture.

### EXAMPLE 10

The in-vitro Microsomal Assay was conducted on compounds listed in Table 10. The results that were obtained are summarized in Table 10. The compounds in Table 10 were tested at 100 µM concentration.

**TABLE 10**

| **COMPOUND** | **% INHIBITION, ARAT** | **% INHIBITION, LRAT** |
|---|---|---|
| Control | 0 | 0 |
| Naringenin | 33 | 14 |
| Quercetin | 25 | 14 |

### EXAMPLE 11

### Naringenin and Retinol Synergistically Inhibit Keratinocyte Differentiation

The effect on TGase I levels normalised to DNA content of the cells was examined in response to a 72 hour treatment with the test compounds. The results are shown in Table 11.
Naringenin was obtained from Sigma.

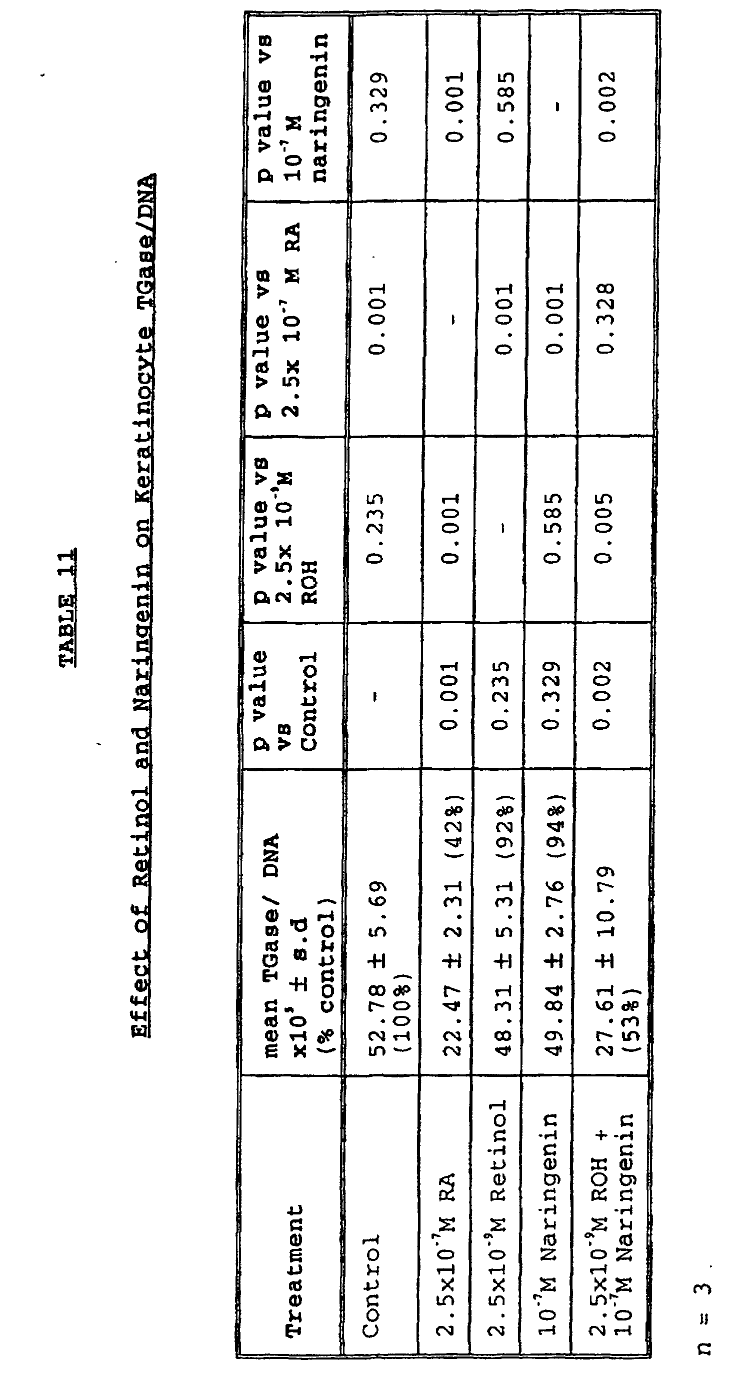

It can be seen from the results in Table 11 that 2.5x10⁻⁷M retinoic acid was very effective at repressing keratinocyte TGase I levels (to 42% of control level). 2.5x10⁻⁹M retinol was ineffective (91%) and 10⁻⁷M naringenin had no inhibitory effect on the keratinocyte TGase I level when used alone. However, 2.5x10⁻⁹M retinol + 10⁻⁷M naringenin repressed keratinocyte TGase I to 53% of control levels. Naringenin and retinol therefore acted synergistically to repress keratinocyte differentiation in an analogous manner to the effect of retinoic acid.

### EXAMPLE 12

### Naringenin and Retinyl Palmitate Synergistically Inhibit Keratinocyte Differentiation

The effect on TGase I levels normalised to DNA content of the cells was examined in response to a 72 hour treatment with the test compounds. The results are shown in Table 12.

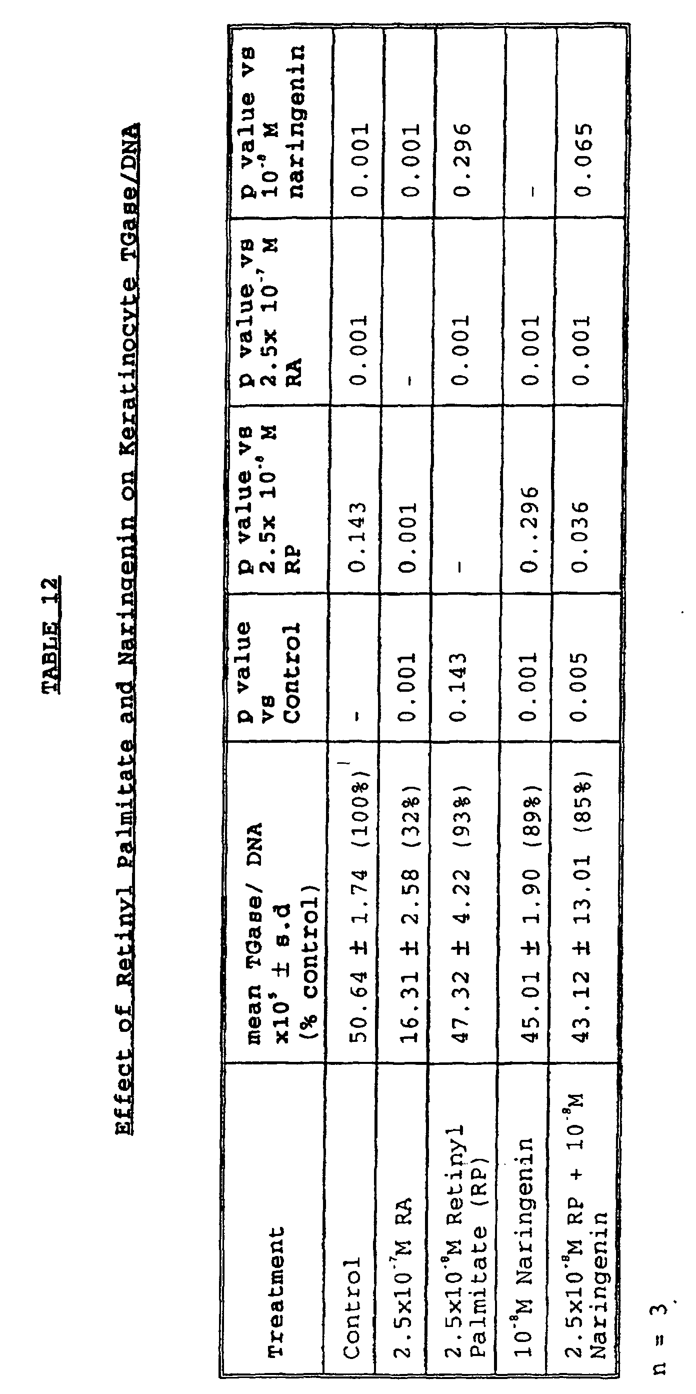

It can be seen from the results in Table 12 that 2.5x10⁻⁷M retinoic acid was very effective at repressing keratinocyte TGase I levels (to 32% of control level). 2.5x10⁻⁹M retinyl palmitate was ineffective (93%) and 10⁻⁹M naringenin had a small inhibitory effect on the keratinocyte TGase I level when used alone. However, 2.5x10⁻⁹M retinol + 10⁻⁹M naringenin repressed keratinocyte TGase I to 85% of control levels. Naringenin and retinyl palmitate therefore act synergistically to repress keratinocyte differentiation in an analogous manner to the effect of retinoic acid.

### EXAMPLE 13

### Quercetin and Retinol Synergistically Inhibit Keratinocyte Differentiation

The effect on TGase I levels normalised to DNA content of the cells was examined in response to a 72 hour treatment with the test compounds. The results are shown in Table 13. Quercetin was obtained from Sigma.

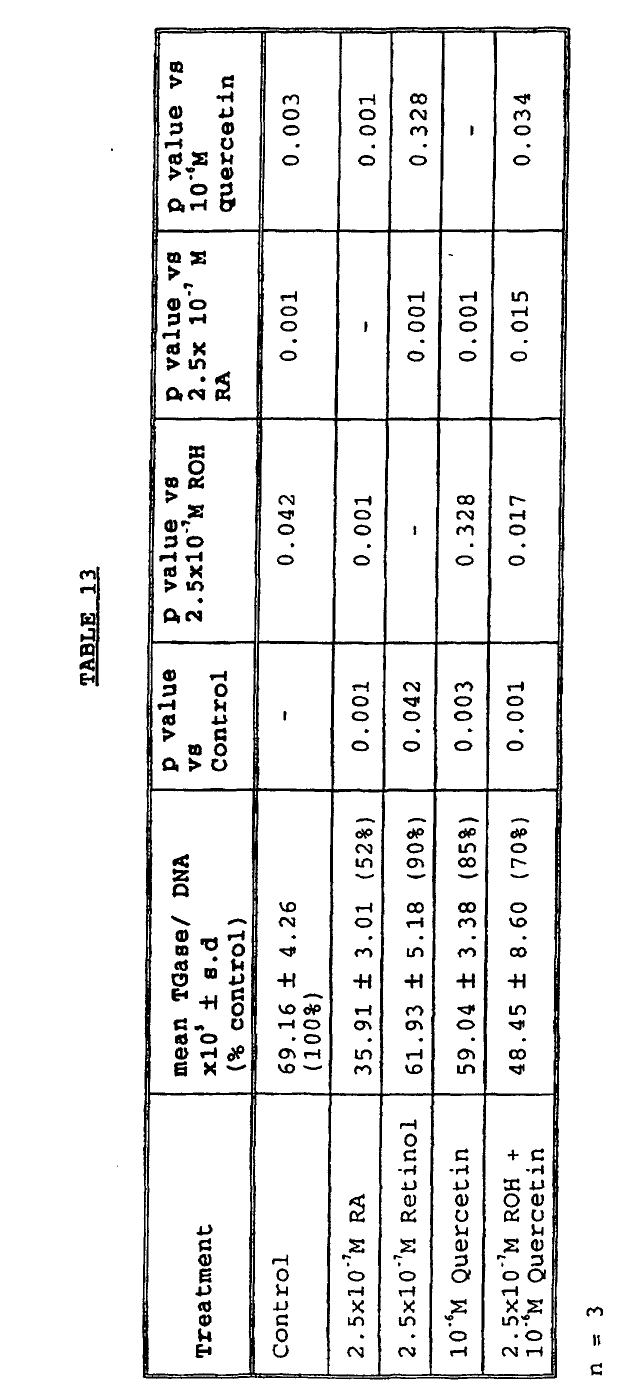

It can be seen from the results in Table 13 that 2.5x10⁻⁷M retinoic acid was effective at repressing keratinocyte TGase I levels (to 52% of control level). 2.5x10⁻⁷M retinol was ineffective (90%) and 10⁻⁶M quercetin had only a small inhibitory effect on the keratinocyte TGase I level when used alone. However, 2.5x10⁻⁷M retinol + 10⁻⁶M quercetin repressed keratinocyte TGase I to 70% of control levels. Quercetin and retinol therefore acted synergistically to repress keratinocyte differentiation in an analogous manner to the effect of retinoic acid.

During the course of these studies, retinoic acid was used as positive control and reference compound against which the other compounds under analysis were compared. Retinoic acid, in a dose dependant manner decreased transglutaminase I levels in skin keratinocytes. In other words, retinoic acid decreased keratinocyte differentiation. Retinol and retinyl palmitate were significantly less effective than retinoic acid at inhibiting keratinocyte differentiation.

The unexpected result demonstrated by the Examples above however was that the effect of retinol or retinyl ester on cultured keratinocytes can be enhanced to levels approaching those of retinoic acid by combining retinol or the ester with a compound which is from 0.0001% to 50% of a compound which at 100 µM concentration inhibits at least 20% of LRAT or ARAT catalyzed retinol esterification as measured by the in vitro Microsomal Assay. This effect was not only greater than the effect of either retinol or the ester or the compound itself but the two ingredients acted in synergy with each other to promote a retinoic acid-type response on the keratinocytes.

The results documented above demonstrate that compounds which pass the in-vitro Microsomal Assay act synergistically with retinol and retinyl esters to decrease keratinocyte differentiation and/or to increase keratinocyte proliferation, mimicking the effect on keratinocytes of retinoic acid.

Examples 14-19 illustrate topical compositions according to the present invention. The compositions can be processed in conventional manner. They are suitable for cosmetic use.
In particular the compositions are suitable for application to wrinkled, rough, dry, flaky, aged and/or UV-damaged skin to improve the appearance and the feel thereof as well as for application to healthy skin to prevent or retard deterioration thereof.

### EXAMPLE 14

This example illustrates high internal phase water-in-oil emulsions incorporating the inventive composition.

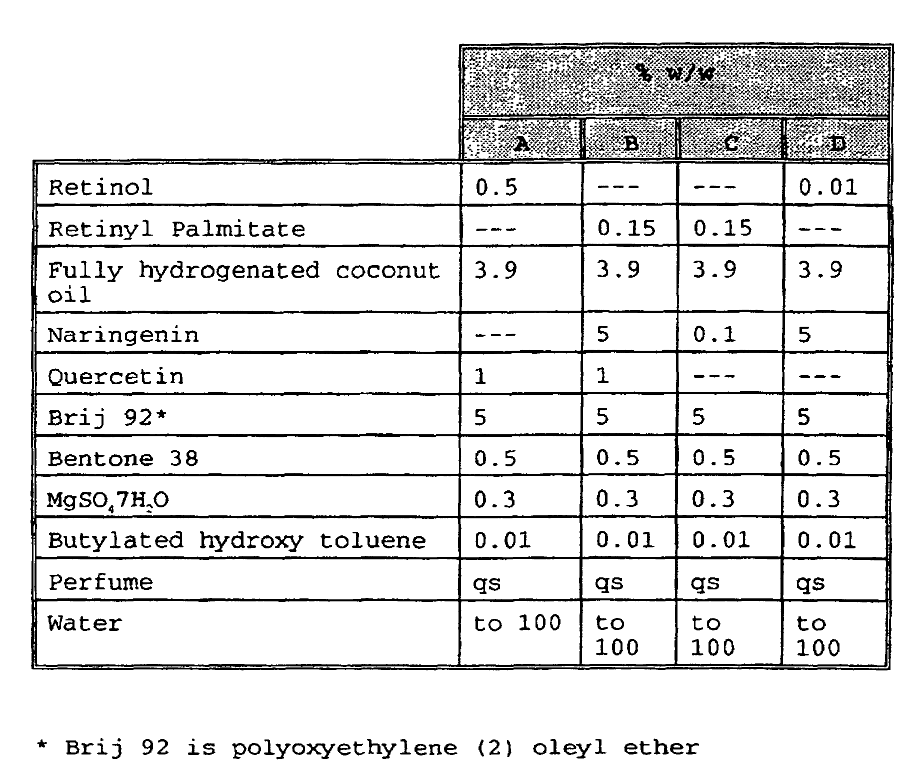

### EXAMPLE 15

This example illustrates oil-in-water creams according to the invention.

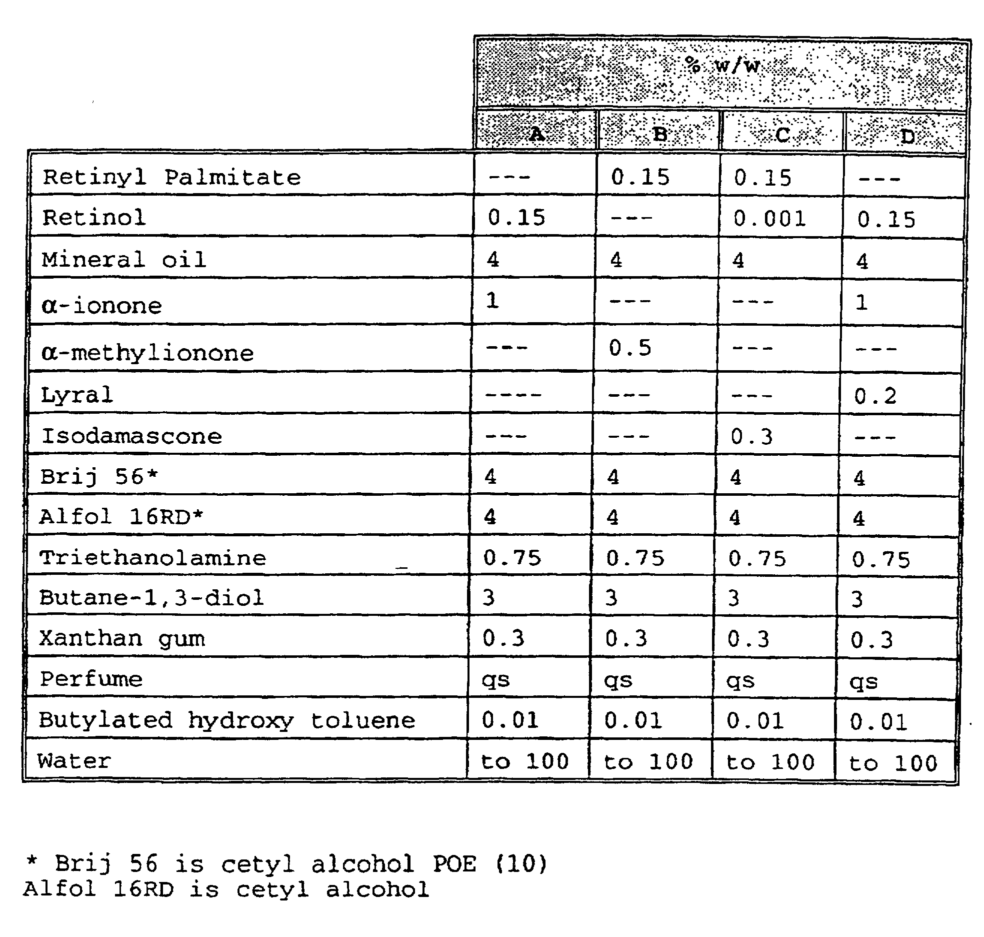

### EXAMPLE 16

This example illustrates alcoholic lotions according to the invention.

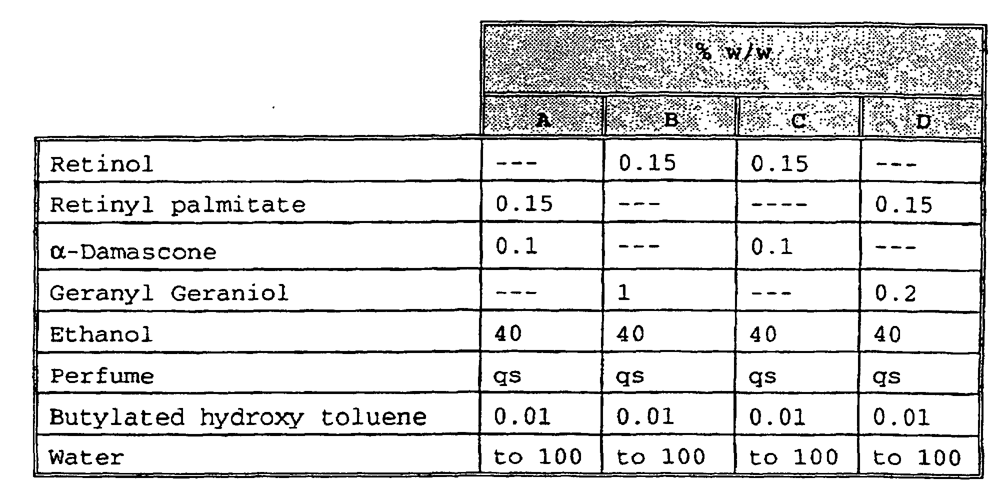

### EXAMPLE 17

This example illustrates another alcoholic lotion containing the inventive composition.

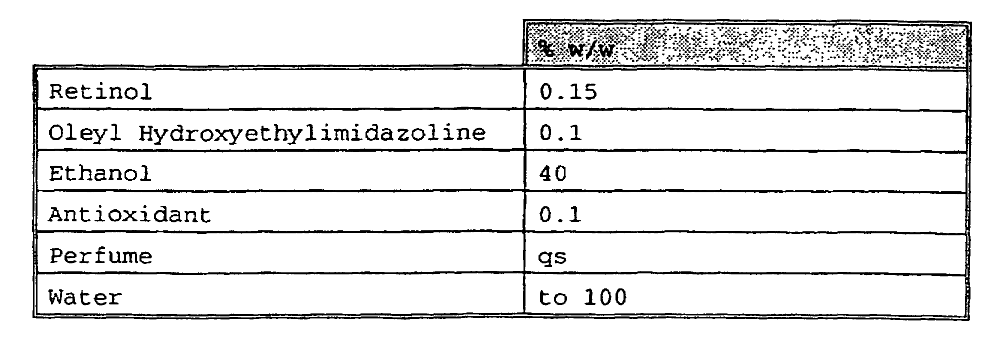

### EXAMPLE 18

This example illustrates a suncare cream incorporating the composition of the invention:

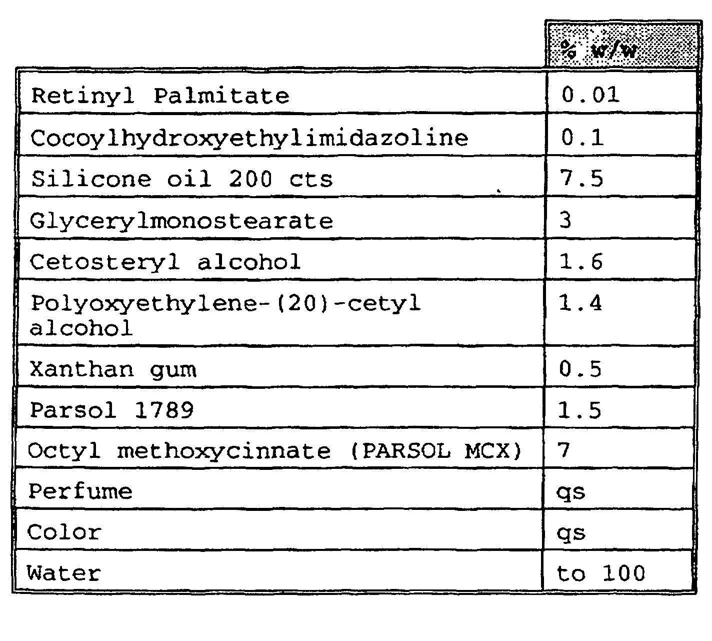

### EXAMPLE 19

This example illustrates a non-aqueous skin care composition incorporating the inventive combination.

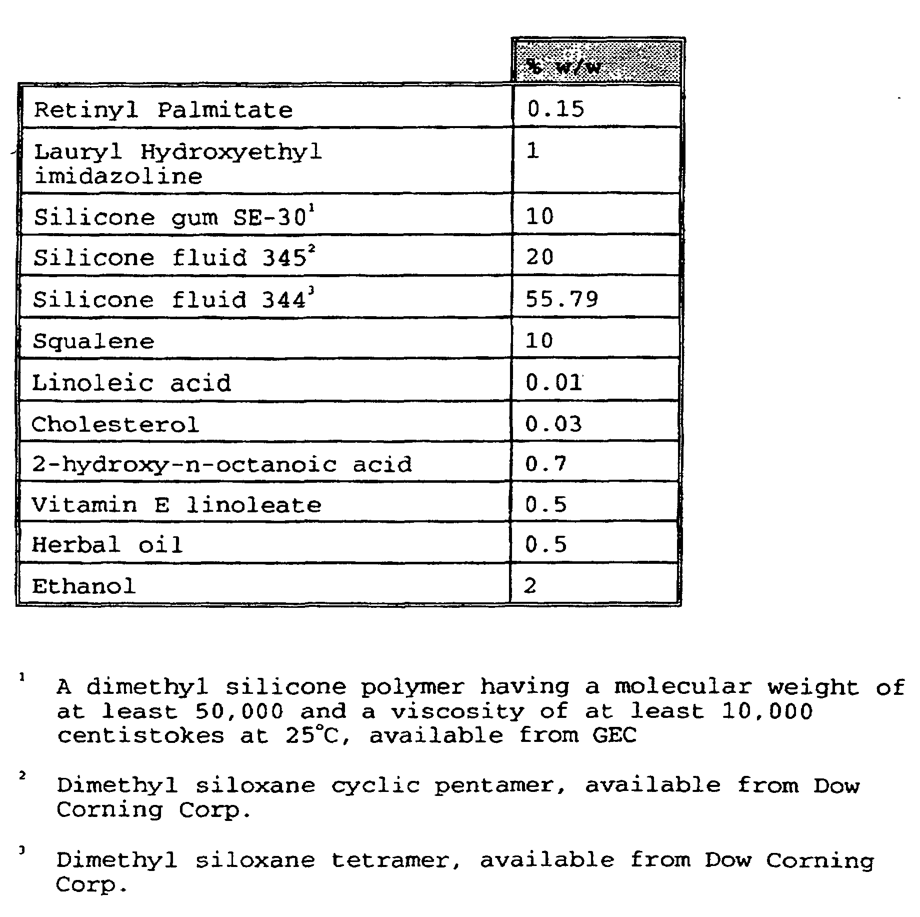

If not mentioned in the Examples, materials employed in the present invention are obtained from the following sources:

Palmitoyl CoA, BSA, dilauroylphospatidyl choline, retinol, retinoic acid dithiothreitol - from Sigma.
Cyclic aliphatic unsaturated compounds:
   Damascones from Firmenich;
   Ionones from IFF;
   Others from suppliers in "Flavor and Fragrance
   Materials" 1991, by Allured Pub. Co.
Imidazoline surfactants:
   The oleyl imidazoline was Schercozoline O from Scher Chemical Co.; The others were from McIntyre, under the Mackazoline name, as follows: Caprylic, Coco, Lauryl: Mackazoline CY, C, and L, respectively.

## Claims

1. A Skin care composition comprising:
(a) from 0.001% to 10% of a compound selected from the group consisting of retinol, retinyl ester and mixtures thereof;
(b) from 0.0001% to 50% of a compound which at 100 µM concentration inhibits at least 20% of LRAT or ARAT catalyzed retinol esterification as measured by an in vitro Microsomal Assay as described herein; wherein the compound is not a fatty acid amide or dimethyl imidazolidinone and is selected from the group consisting of cyclic aliphatic unsaturated hydrocarbons, diterpenes, and fatty hydroxyethyl imidazoline surfactants having the following structure: 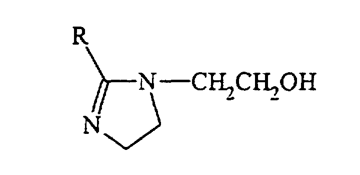 wherein R is an aliphatic saturated or unsaturated, straight or branched hydro-carbon chain containing from 8 to 20 carbon atoms; and mixtures thereof; and
(c) a cosmetically acceptable vehicle.

2. The composition of claim 1 wherein the cyclic aliphatic unsaturated compound is selected from the group consisting of alpha damascone, beta damascone, delta damascone, isodamascone, damascenone, alpha ionone, beta ionone, allyl alpha ionone, isobutyl ionone, alpha methyl ionone, gamma methyl ionone, brahmanol, sandanol, alpha terpineol, lyral, ethyl saffranate, and mixtures thereof.

3. The composition of claim 1 wherein the diterpene is geranyl geraniol.

4. The composition according to any one of claims 1-4 wherein the retinyl ester is selected from the group consisting of retinyl palmitate, retinyl acetate, retinyl propionate, retinyl linoleate and mixtures thereof.

5. A cosmetic method of conditioning skin the method comprising applying topically to skin a cosmetic composition according to any one of claims 1-4.

6. A cosmetic method of conditioning skin by mimicking the effect of retinoic acid, the method comprising applying to the skin the cosmetic composition according to any one of claims 1-4.

## Patentansprüche

1. Hautpflegezusammensetzung, umfassend:
a) 0,001% bis 10% einer Verbindung, ausgewählt aus der Gruppe, bestehend aus Retinol, Retinylester und Gemischen davon;
b) 0,0001% bis 50% einer Verbindung, die bei einer Konzentration von 100 µM mindestens 20% einer LRAT oder ARAT katalysierten Retinolveresterung, wie durch ein hierin beschriebenes mikrosomales in-vitro-Assay gemessen, inhibiert; wobei die Verbindung kein Fettsäureamid oder Dimethylimidazolidinon darstellt und aus der Gruppe, bestehend aus cyclischen aliphatischen ungesättigten Kohlenwasserstoffen, Diterpenen und Fettsäurehydroxyethylimidazolintensiden der nachstehenden Struktur: 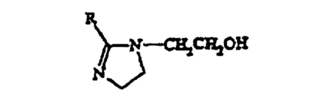 worin R eine aliphatische gesättigte oder ungesättigte gerade oder verzweigte Kohlenwasserstoffkette mit 8 bis 20 Kohlenstoffatomen darstellt und Gemischen, davon ausgewählt ist und
c) einen kosmetisch verträglichen Träger.

2. Zusammensetzung nach Anspruch 1, worin die cyclische aliphatische ungesättigte Verbindung aus der Gruppe, bestehend aus α-Damascon, β-Damascon, δ-Damascon, Isodamascon, Damascenon, α-Jonon, β-Jonon/ Allyl-α-jonon, Isobutyljonon, α-Methyljonon, γ-Methyljonon, Brahmanol, Sandanol, α-Terpineol, Lyral, Safransäureethylester und Gemischen davon ausgewählt ist.

3. Zusammensetzung nach Anspruch 1, worin das Diterpen Geranylgeraniol darstellt.

4. Zusammensetzung nach einem der Ansprüche 1 bis 4, worin der Retinylester aus der Gruppe, bestehend aus Palmitinsäureretinylester, Essigsäureretinylester, Propionsäureretinylester, Linolsäureretinylester und Gemischen davon, ausgewählt ist.

5. Kosmetisches Verfahren zum Konditionieren von Haut, wobei das Verfahren Auftragen einer kosmetischen Zusammensetzung nach einem der Ansprüche 1 bis 4 örtlich auf die Haut umfasst.

6. Kosmetisches Verfahren zum Konditionieren von Haut durch Nachahmen der Wirkung von Retinsäure, wobei das Verfahren Auftragen der kosmetischen Zusammensetzung nach einem der Ansprüche 1 bis 4 auf die Haut umfasst.

## Revendications

1. Composition pour le soin de la peau, comprenant :
(a) de 0,001 % à 10 % d'un composé sélectionné à partir du groupe constitué du rétinol, de l'ester de rétinyle et des mélanges de ceux-ci ;
(b) de 0,0001 % à 50 % d'un composé qui, a une concentration de 100 µM, inhibe au moins 20 % de l'estérification du rétinol catalysé LRAT ou ARAT, comme mesuré par un Essai Micromosal in vitro tel que décrit aux présentes ; dans lequel le composé n'est pas un amide d'acide gras ou un imidazolidinone de diméthyle et est sélectionné à partir du groupe constitué des hydrocarbures insaturés aliphatiques cycliques, des dipertènes et des tensioactifs imidazoline d'hydroxyéthyle gras ayant la structure suivante : 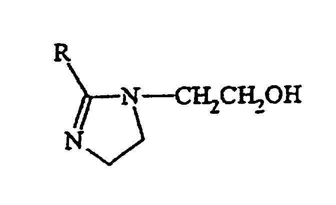 dans laquelle R est une chaîne hydrocarbonée aliphatique saturée ou insaturée, linéaire ou ramifiée contenant de 8 à 20 atomes de carbone ; et des mélanges de ceux-ci ; et
(c) un matériau formant support acceptable d'un point de vue cosmétique.

2. Composition selon la revendication 1, dans laquelle le composé insaturé aliphatique cyclique est sélectionné à partir du groupe constitué de l'alpha damascone, du bêta damascone, du delta damascone, de l'isodamascone, de l'alpha ionone, du delta ionone, de l'alpha ionone d'allyle, de l'ionone d'isobutyle, de l'alpha méthyle ionone, du gamma méthyl ionone, du brahmanol, du sandanol, de l'alpha terpinol, du lyral, du saffranate d'éthyle et des mélanges de ceux-ci.

3. Composition selon la revendication 1, dans laquelle le dipertène est du géraniol de géranyle.

4. Composition selon l'une quelconque des revendications 1 - 4, dans laquelle l'ester de rétinyle est sélectionné à partir du groupe constitué du palmitate de rétinyle, de l'acétate de rétinyle, du propionate de rétinyle, du linoléate de rétinyle et des mélanges de ceux-ci.

5. Procédé cosmétique de conditionnement de la peau, ledit procédé comprenant le fait d'appliquer sur la peau une composition cosmétique selon l'une quelconque des revendications 1 - 4.

6. Procédé cosmétique de conditionnement de la peau par imitation de l'effet de l'acide rétinoïque, ledit procédé comprenant le fait d'appliquer sur la peau la composition cosmétique selon l'une quelconque des revendications 1 - 4.
